# EUROPÄISCHE PATENTSCHRIFT

(11) **EP 1 357 859 B1**
(45) Veröffentlichungstag und Bekanntmachung des Hinweises auf die Patenterteilung: **21.05.2008**
(21) Anmeldenummer: 02711822.3
(22) Anmeldetag: 30.01.2002
(51) Int. Cl.: A61F 2/06, A61B 17/11

(54) **KOMBINATION AUS GEFÄSSPROTHESE UND HALTEELEMENT**
COMBINATION OF A VASCULAR PROSTHESIS AND RETAINING ELEMENT
COMBINAISON D'UNE ENDOPROTHESE VASCULAIRE A UN ELEMENT DE MAINTIEN

(30) Priorität: 01.02.2001 DE 10104361
(43) Veröffentlichungstag der Anmeldung: 05.11.2003
(73) Patentinhaber: Zühlke, Helmut, 06886 Lutherstadt Wittenberg (DE)
(72) Erfinder: Zühlke, Helmut, 06886 Lutherstadt Wittenberg (DE)
(74) Vertreter: Thiel, Christian
(86) Internationale Anmeldenummer: PCT/EP2002/000941
(87) Internationale Veröffentlichungsnummer: WO 2002/060348

(56) Entgegenhaltungen:
- EP-A- 0 712 614
- EP-A- 0 732 088
- EP-A- 1 055 401
- WO-A-00/61034
- WO-A-98/19631
- US-A- 5 591 226
- US-A- 5 676 670
- US-A- 6 152 945

## Beschreibung

Die Erfindung betrifft eine Kombination einer Gefäßprothese mit einem Halteelement, welche insbesondere bei der Behandlung chronischer Verschlußerkrankungen zum Einsatz kommt.

Die bei der Behandlung chronisch arterieller Verschlußerkrankungen herkömmlich angewandten Bypassverfahren mit Hilfe künstlicher oder körpereigener Ersatzgefäße sind aufgrund der hohen Invasivität mit einem hohen Infektionsrisiko verbunde. Als Alternative zum Bypassverfahren kommt daher oftmals die sogenannte Ausschälplastik zum Einsatz, bei der das Gefäß proximal des zu behandelnden Areals eröffnet wird und der auszuschälende Verschlußzylinder zirkulär mit einem feinen spatelähnlichen Instrument isoliert und durchtrennt wird. Über den sich nach distal erstreckenden Intimazylinder wird ein Ringstripper gefädelt, welcher unter Anspannung des Intimadissektates zirkulär unter Anwendung leichten Drucks nach peripher vorgeschoben wird. Am Ende des stenotischen Bereichs wird das Dissektat mit einem sich im Ringstripper befindenden Seil durchtrennt, Zwar ist hier das Infektionsrisiko gegenüber dem Bypassverfahren vermindert, jedoch kommt es in dem ausgeschälten (desobliterierten) Gefäß ofmals zur erneuten Bildung eines Intimazylinders (Intimahyperplasie), was langfristig ein erneutes operatives Vorgehen nötig macht.

Eine weitere Möglichkeit der Behandlung stenotischer Bereiche, ist der Einsatz von Stents (Platzhalter, Gefäßstützen). Diese aus verschiedenen Materialien bekannten Vorrichtungen, werden in den stenötischen Gefäßbereich eingebracht, unter Anwendung großer Drücke aufgedehnt und dienen dazu, die Gefäße dauerhaft offen zu halten. Nachteil ist jedoch, daß dabei der verformte Intimazylinder im Gefäßsystem verbleibt. Aufgrund der starken mechanischen Belastung bei der Anlegung und Aufweitung des Stents kommt es daher oftmals zu einer dehnungs- und verletzungsinduzierten Hyperplasie der Intima an den unmittelbar angegrenzenden Bereichen.

Es besteht somit Bedarf an Vorrichtungen, welche die Behandlung arterieller Verschlußerkrankungen erlauben, ohne die mit dem Stand der Technik verbundenen Nachteile aufzuweisen.

US 5,591,226 A beschreibt eine Stent-Implantat-Kombination, bei welcher das schlauchförmige Implantat entweder über die gesamte Länge oder zumindest an beiden Enden mit Stents zur Verankerung in einem Gefäß versehen ist.

Die beiden Enden können zusätzlich eingenäht werden.

Diese Aufgabe wird erfindungsgemäß gelöst durch eine Kombination einer Gefäßendoprothese und Halteelement gemäß Anspruch 1.

Die erfindungsgemäße Kombination wird an die Einsatzstelle geführt, wo das röhrenförmige Halteelement nach Aufweitung die Gefäßprothese von intraluminal in dem zu behandelnden Gefäß verspannt. Die erfindungsgemäße Kombination kann grundsätzlich in jedem Körperbereich zur Anwendung kommen, in dem ihre Platzierung chirurgisch möglich ist. Vorzugsweise wird sie bei der Behandlung chronisch arterieller Verschlußerkrankungen der großen Leitungsbahnen, insbesondere in den Extremitäten und der Beckenetage zur Anwendung kommen.

Als röhrenförmiges Halteelement wird dabei vorzugsweise ein Halteelement eingesetzt, dessen Aufbau und Material dem von Gefäßstützen (Stents, Platzhaltern) zumindest ähnlich ist. Das Halteelement kann dabei zumindest teilweise in der Wandung der Gefäßprothese angeordnet sein; vorzugsweise ist das Halteelement so angeordnet, daß es zumindest teilweise in das Innenlumen der Gefäßprothese ragt. In dieser bevorzugten Ausführungsform ist die Verspannung der Gefäßprothese durch das Halteelement besonders stabil. Überdies kann die erfindungsgemäße Kombination in dieser Ausführungsform einfach aus Einzelkomponenten (Halteelement und Gefäßprothese) zusammengesetzt werden.

Das Halteelement kann dabei als internes Halteelement gänzlich in der Gefäßprothese angeordnet sein. In dieser Ausführungsform ist an der erfindungsgemäßen Kombination vorzugsweise ein weiteres Halteelement außen an der Gefäßprothese angeordnet (externes Halteelement), so daß ein nahtloser Übergang von der Gefäßprothese zur Innenwandung des behandelten Gefäßes geschaffen wird.

Gemäß einer weiteren vorteilhaften Ausführungsform ist das Halteelement so angeordnet, daß es nur teilweise in die Gefäßprothese ragt und teilweise (d. h. über das distale Ende der Gefäßprothese hinweg) aus der Gefäßprothese herausragt. In dieser besonders bevorzugten Ausführungsform ist ein einziges Halteelement ausreichend, um die Verspannung, der Gefäßprothese im zu behandelnden Gefäß und den gleichmäßige Übergang von der Gefäßprothese zur Innenwandung gleichermaßen zu ermöglichen.

Besonders bevorzugt ist eine Kombination, in der das Halteelement zu über 50 % seiner Länge innerhalb und zu unter 50 % seiner Länge außerhalb der Prothese angeordnet ist, so daß eine besonders gute Verspannung der Gefäßprothese gewährleistet ist.

Gemäß der Erfindung sind nur an einem Ende der Gefäßprothese ein oder mehrere Halteelemente angeordnet. In dieser Ausführungsform wird die erfindungsgemäße Kombination mittels eines Katheters endoskopisch nach distal zur verbleibenden Intimastufe geführt. Nach Aufweitung des Halteelements wird der Katheter zurückgezogen. Die Aufweitung kann dabei durch den Katheter erfolgen (mittels eines Ballonkatheters z.B.). Zweckmäßig ist auch die Verwendung von selbstexpandierenden Halteelementen, die komprimiert eingeführt werden und sich nach Wegfall eines von außen einwirkenden Zwanges (z. B. durch den Katheter oder das Gefäß) selbst aufweitet. Am proximalen Ende wird die Gefäßprothese auf herkömmliche Weise mit dem zu behandelnden Gefäß vernäht. Der Vorteil dieser Ausführungsform liegt in der besonderen Stabilität der Fixierung der Gefäßprothese bei minimierter Invasivität und somit verringertem Infektionsrisiko. Die Anordnung der erfindungsgemäßen Kombination erfolgt dabei strömungstechnisch so, daß das Halteelement in Blutflußrichtung distal angeordnet ist.

Zweckmäßig ist ebenso die feste Verbindung der Gefäßprothese mit dem oder den Halteelementen, so daß diese bei der Einbringung oder danach nicht verrutschen. Dies kann z. B. durch Verkleben, Löten, Schweißen oder andere, dem Fachmann bekannte Techniken erfolgen.

Gemäß der Erfindung ist die Gefäßprothese eine endoluminale Gefäßprothese. In dieser Ausführungsform eignet sich die erfindungsgemäße Kombination zur Behandlung chronischer Verschtußerkrankungen mittels extra-/endovaskulärer Hybridtechniken, wobei das Halteelement distal zur Verspannung der Gefäßendoprothese bei gleichzeitiger Dilatation des Gefäß und Fixierung der Intimastufe dient. Die Gefäßendoprothese dient zur Verhinderung der erneuten Bildung und Hyperproliferation einer Intima dort, wo das Intimadissektat obliteriert wurde.

Die Gefäßprothese besteht dabei vorzugsweise aus PTFE (Polytetrafluorethylen) oder Doppeldacronvelour oder anderen Materialien die aufgrund ihrer guten Stabilität und Elastizität die Ausbildung möglichst dünnwandiger Gefäßprothesen ermöglichen. Die Gefäßprothese weist dabei einen Durchmesser auf, welcher an den Lumendurchmesser des jeweiligen Gefäßes angepaßt ist. Sie kann ein im Querdurchmesser gleichbleibenden Durchmesser und/oder Lumen aufweisen, aber auch konisch ausgeformt sein und sich so an die anatomische Verengung von Gefäßen im Verlaufe von proximal nach distal anpassen.

Die verwendeten Halteelemente sind vorzugsweise komprimierbar und aus einem oder mehreren Materiallen gefertigt, welche nach Expansion stabil bleiben und vorzugsweise ein sogenanntes "Form-Gedächnis" aufweisen. Als Materialien eignen sich dabei neben Kunststoffen insbesondere Metalle wie Eisen, Tantal, Titan, Niob, Platin und Legierungen aus mindestens einem dieser Metalle mit mindestens einem weiteren Metall und insbesondere Nitinol. (Dieses Material ist besonders geeignet, weil sich daraus selbstexpandierende Halteelemente mit Formgedächtnis herstellen lassen.) Die Halteelemente können darüber hinaus mit biokompatiblen Beschichtungen versehen sein (insbesondere dort, wo sie mit der Intima des behandelten Gefäßes in Kontakt kommen).

Gemäß einer besonders zweckmäßigen Ausführungsform werden als Halteelemente Stents eingesetzt. In dieser Ausführungsform kann die erfindungsgemäße Kombination technisch einfach aus verschiedenen, kommerziell einzeln erhältlichen Teilen (Prothese und Stents) zusammengesetzt werden. Als Stents können dabei z. B. solche eingesetzt werden, die durch Einwirkung eines Druckes von ihrem Lumen her (z. B. durch einen Ballonkatheter) aufgeweitet werden. Zweckmäßig ist auch der Einsatz von selbstexpandierenden Stents.

Besonders bevorzugt ist eine Ausführungsform, in der ein Halteelement (vorzugsweise ein Stent) so an einem Ende der Gefäßendoprothese fixiert ist, daß es teilweise (vorzugsweise zu über 50 % seiner Länge) in das Innenlumen und teilweise (vorzugsweise zu unter 50 % seiner Länge) aus diesem nach distal herausragt.

Die Erfindung wird nachfolgend anhand einer Figur und eines Ausführungsbeispiels näher erläutert.

Es stellt dar:

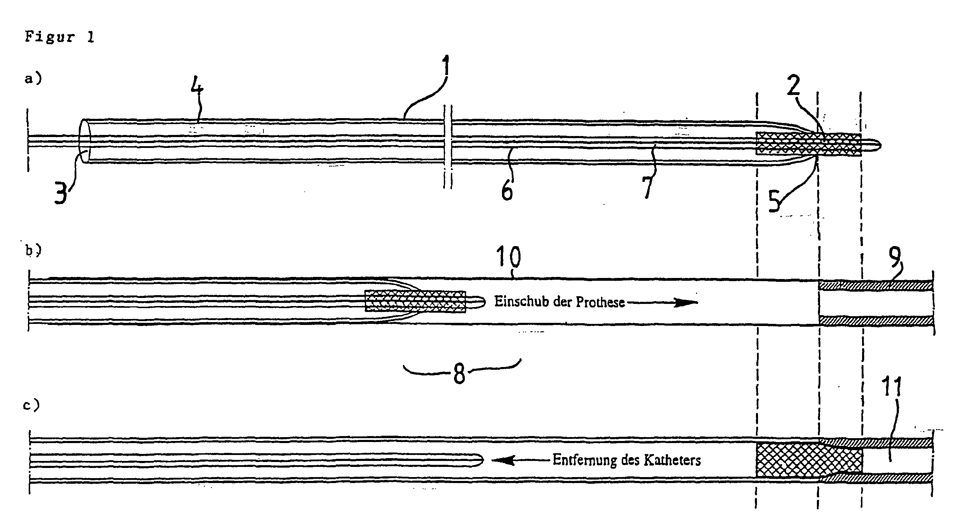

- Figur 1 (a, b, c): einen Längsschnitt durch eine Kombination aus Gefäßendoprothese und einem Stent in Seitenansicht.

Die in der Figur dargestellte Kombination besteht aus einer Gefäßendoprothese 1, an deren einem Ende als Halteelement ein Stent 2 so angeordnet ist, daß er zu mehr als der Hälfte in dem Innenlumen 3 des Prothesenrohrs 4 angeordnet ist und zu weniger als der Hälfte über das distale Ende 5 der Gefäßendoprothese 1 nach außen herausragt. Der Stent 2 ist als hochflexibles Biegeelement aus Nitinol gefertigt. Die Gesamtlänge der Gefäßendoprothese beträgt ca. 40 cm.

Wie in Figur 1b dargestellt, wird die Kombination mit Hilfe eines Einführkatheters 6 (mit einem Innenlumen 7 für einen Mandrin oder zur Aufnahme einer Spüllösung) endoskopisch in dem teil-desobliterierten (d. h. streckenweise ausgeschälten) Gefäß 8 bis zur verbleibenden Intimastufe 9 vorgeschoben. Die Intimastufe 9 (Intimalefze) wird durch die an den desobliterierten Bereich angrenzende, verbleibende Intima des Blutgefäßes 8 gebildet. Der Einführkatheter 6 ist als Ballonkatheter ausgebildet, auf dem der Stent 2 von außen aufgesetzt ist. Nach Erreichen der Intimastufe wird die Kombination so angeordnet, daß die Gefäßendoprothese 1 proximal an die Intimastufe 9 angrenzt. Durch Aufweiten des Ballonkatheters 6 wird der Stent 2 radial expandiert und verspannt so die Gefäßendoprothese 1 mit der verbliebenen Adventitia 10 des desobliterierten Gefäße 8 und stellt gleichzeitig einen gleichmäßigen Übergang des Innenlumens der Gefäßendoprothese 1 zum Innenlumen des behandelten Gefäßes 11 her.

Anschließend wird der Ballonkatheter 6 wieder komprimiert und nach proximal aus dem Gefäß entfernt. In dem Gefäß verbleibt die Kombination aus Gefäßendoprothese 1 und nunmehr radial expandierten Stent 2, wobei das Prothesenrohr 4 sich intraluminal über die Adventitia fügt und eine Neubildung einer Intima verhindert. Die dichte Verspannung des distalen Endes 5 der Gefäßendoprothese 1 durch den radial expandierten Stent 2 in dem behandelten Gefäß verhindert gleichzeitig ein Einwachsen der Muskelzellen der verbliebenen Intimastufe 9 auf die Adventitia 10 des desobliterierten Bereichs des behandelten Gefäßes 8.

### Beispiel 1:

Extra-endoluminale Implantation einer Kombination aus Gefäßendoprothese und Stent bei chronisch-arterieller Verschlußerkrankung (paVK) im Bereich der Arteria femoralis superficialis.

Nach Freilegung der Gefäße im Bereich der Leiste (A. femoralis communis, A. profunda femoris und A. femoralis superficialis) wird das Gefäßsystem über dem Abgang der A. profunda femoris eröffnet und der Schnitt in die A. femoralis superficialis (Leitungsarterie zum Unterschenkel) verlängern.

Dicht hinter dem Abgang der A. femoralis superficialis wird der Intimazylinder ausgeschält, zirkulär freigemacht, durchtrennt und mit einem Ringstripper bis in Höhe der A. poplitea desobliteriert. (Ausführung des Adduktorenkanals, auch als Empfängersegment bezeichnet, da hier die Kollateralgefäße der A. profunda femoris einstrahlen.) Dieses Vorgehen wird unter röntgenologischer Kontrolle durchgeführt.

Nach Entfernung des Intimazylinders wird das offene Lumen der A. poplitea sondiert und ein Führungsdraht in die A. poplitea ins II. und III. Segment vorgeschoben. Über den Führungsdraht wird dann ein dickerer Führungsdraht eingelegt. Nach weiterer röntgenologischer Kontrolle wird dann das aus Gefäßendoprothese und Stent bestehende Prothesensystem (Device) eingebracht. Mittels eines Ballon-Katheters, auf dem der Stent zusammengefaltet aufgesetzt ist, wird das Prothesensystem nach distal vorgeschoben. Der nach außen aus der Gefäßendoprothese ragende Anteil des Stents wird über die Intimalefze (verbleibende Muskelschicht) geschoben und anschließend der gesamte Stent mit Hilfe des Ballons aufgedehnt. Unter röntgenologischer Kontrolle werden im Bereich des Überganges der desobliterierten A. femoralis superficialis und der A. poplitea mit Hilfe des Ballons der Stent zusammen mit der Gefäßendoprothese entfaltet und anschließend der Katheter zurückgezogen, wobei die Prothese von dem Katheter abgestreift wird. Der Katheter wird dann zur Leistenregion hin hochgezogen. Die Gefäßendoprothese ist durch den intraluminalen Stent im Gefäß fixiert. Das Gefäß selbst wird an dieser Stelle zusätzlich noch etwas dilatiert. Gleichzeitig wird die Intimastufe ebenfalls durch die Stents fixiert.

Der Katheter wird nach intravasaler Fixierung des distalen Endes der Gefäßendoprothese durch Aufdehnung des Stents, wobei eine möglichst dünnwandige Prothese (entweder PTFE oder Doppeldacronvelour) verwandt werden kann, zurückgezogen. Die Gefäßendoprothese kann in der gesamten Länge eine gleichbleibendes Lumen mit Durchmesser von 6 bis 8 mm besitzen; es kann auch eine konische Prothese, von 6 auf 8 mm zulaufend, verwendet werden.

Im Bereich der Arteriotomie wird in der Leistenregion die Prothese zurechtgeschnitten und in konventioneller Nahttechnik in die A. femoralis superficialis eingenäht, wobei die Hinterwand in transluminaler Technik angelegt werden nuß, während die Vorderwand in typischer Technik in die eröffnete A. femoralis communis eingenäht werden kann. Zusätzlich kann gegebenenfalls eine Erweiterungsplastik der A. femoralis communis durchgeführt werden. Nach Fertigstellung der Anastomosen wird dann der Blutstrom in die Peripherie freigegeben. Der Vorteil der Methode besteht in der geringeren Invasivität (nur eine Gefäßfreilegung) - damit Verringerung der Infektionsrate und der orthotopen Gefäßrekonstruktion - keine zusätzliche Traumatisierung für ein Implantantlager und dem Zeitgewinn. Die intraluminalen Prothese ist dabei mit einem geringeren Risiko der Hyperplasie verbunden als extraanatomisch plazierte Prothese. Die Technik kann ebenfalls im Bereich der Beckenetage retrograd, d. h. nach Ausschälplastik der A. iliaca externa und A. iliaca communis von der Leiste aus eingesetzt werden.

## Patentansprüche

1. Kombination aus Gefäßendoprothese (1) und Halteelement (2) zur endoskopischen Behandlung von chronische Verschlusskrankheiten, wobei die Gefäßendoprothese (1) ein oder mehrere Halteelemente (2) aufweist und mit dem zu behandelnden Gefäß vemähbar ist, **dadurch gekennzeichnet, dass** die Gefäßendoprothese (1) das oder die Halteelement (2) nur an ihrem distalen Ende aufweist, wobei ein röhrenförmiges Halteelemente (2) zumindest teilweise in die Gefäßendoprothese (1) ragt, und das proximale Ende der Gefäßendoprothese (1) mit dem zu behandelnden Gefäß vernähbar ist.

2. Kombination gemäß Anspruch 1, **dadurch gekennzeichnet, daß** das röhrenförmige Halteelement (2) zumindest teilweise in das Innenlumen der Gefäßendoprothese (1) ragt.

3. Kombination gemäß einem der vorstehenden Ansprüche, **dadurch gekennzeichnet, daß** das röhrenförmige Halteelement (2) gänzlich in der Gefäßendoprothese (1) angeordnet ist (internes Halteelement (2)).

4. Kombination gemäß einem der Ansprüche 1 oder 3, **dadurch gekennzeichnet, daß** an dem distalen Ende ein weiteres Halteelement (2) außen an der Gefäßendoprothese (1) angeordnet ist (externes Halteelement (2)).

5. Kombination gemäß Anspruch 1 oder 2, **dadurch gekennzeichnet, daß** das röhrenförmige Halteelement (2) über das distale Ende hinweg aus der Gefäßendoprothese (1) hinausragt.

6. Kombination gemäß Anspruch 5, **dadurch gekennzeichnet**, das Halteelement zu über 50 % innerhalb der Gefäßendoprothese (1) angeordnet ist.

7. Kombination gemäß einem der vorstehenden Ansprüche, **dadurch gekennzeichnet, daß** die Gefäßendoprothese (1) mit dem oder den Halteelementen (2) fest verbunden ist.

8. Kombination gemäß einem der vorstehenden Ansprüche, **dadurch gekennzeichnet, daß** die Gefäßendoprothese (1) aus PTFE oder Doppeldacronvelour besteht.

9. Kombination gemäß einem der vorstehenden Ansprüche, **dadurch gekennzeichnet, daß** die Gefäßendoprothese (1) konisch ausgeformt ist.

10. Kombination gemäß einem der vorstehenden Ansprüche, **dadurch gekennzeichnet, daß** das oder die internen und/oder externen Halteelemente (2) komprimierbar und radial expandierbar sind.

11. Kombination gemäß einem der vorstehenden Ansprüche, **dadurch gekennzeichnet, daß** das oder die Halteelemente (2) im wesentlichen aus einem oder mehreren Kunststoffen und/oder einem oder mehreren Metallen der Gruppe Eisen, Tantal, Titan, Niob, Platin oder einer Legierung aus mindestens einem dieser Metalle mit mindestens einem weiteren Metall und vorzugsweise aus Nitinol gefertigt sind.

12. Kombination gemäß einem der vorstehenden Ansprüche, **dadurch gekennzeichnet, daß** mindestens eines der Halteelemente ein Stent (2) Ist.

## Claims

1. Combination of a vascular endoprosthesis (1) and a retaining element (2) for the endoscopic treatment of chronic occlusion illnesses, the vascular endoprotsthesis (1) having one or more retaining elements (2) and being sewable to the vessel to be treated, **characterized in that** the vascular endoprosthesis (1) comprises the one or more retaining elements (2) only at its distal end, a tubular retaining element (2) projecting at least partially into the vascular endoprosthesis (1), and the proximal end of the endoprosthesis (1) being sewable to the vessel to be treated.

2. The combination according to claim 1, **characterized in that** the tubular retaining element (2) projects at least partially into the inner lumen of the vascular endoprosthesis (1).

3. The combination according to any one of the above claims, **characterized in that** the tubular retaining element (2) is arranged in the vascular endoprosthesis (1) (internal retaining element (2)).

4. The combination according to one of the claims 1 or 3, **characterized in that** another retaining element (2) is arranged at the distal end external to the vascular endoprosthesis (1) (external retaining element (2)).

5. The combination according to claim 1 or 2, **characterized in that** the tubular retaining element (2) projects distally out of the vascular endoprosthesis (1).

6. The combination according to claim 5, **characterized in that** the retaining element is arranged by more than 50 % of its length within the vascular endoprosthesis (1).

7. The combination according to any one of the above claims, **characterized in that** the vascular endoprosthesis (1) is firmly connected with the retaining element(s) (2).

8. The combination according to any one of the above claims, **characterized in that** the vascular endoprosthesis (1) is made of PTFE or double Dacron velour.

9. The combination according to any one of the above claims, **characterized in that** the vascular endoprosthesis (1) has a tapering form.

10. The combination according to any one of the above claims, **characterized in that** the internal and/or external retaining element(s) (2) are compressible and radially expandable.

11. The combination according to any one of the above claims, **characterized in that** the retaining element(s) (2) primarily consist of one or several plastic materials and/or one or several metals of the group iron, tantalum, titanium, niobium, platinum or an alloy consisting of at least one of these metals combined with at least another metal, and, preferably, Nitinol.

12. The combination according to any one of the above claims, **characterized in that** at least one of the retaining elements is a stent (2).

## Revendications

1. Combinaison d'une endoprothèse vasculaire **(1)** et d'un élément de maintien **(2)** pour le traitement endoscopique de maladies vasculaires chroniques, l'endoprothèse vasculaire **(1)** présentant un ou plusieurs éléments de maintien **(2)** et pouvant être cousue au vaisseau à traiter, **caractérisée en ce que** l'endoprothèse vasculaire **(1)** présente le ou les éléments de maintien **(2)** seulement à son extrémité distale, un élément de maintien **(2)** tubulaire formant saillie au moins partiellement dans l'endoprothèse vasculaire **(1)**, et l'extrémité proximale de l'endoprothèse vasculaire **(1)** pouvant être cousue au vaisseau à traiter.

2. Combinaison selon la revendication 1, **caractérisée en ce que** l'élément de maintien tubulaire **(2)** forme saillie au moins partiellement dans l'ouverture intérieure de l'endoprothèse vasculaire **(1)**.

3. Combinaison selon l'une des revendications précédentes, **caractérisée en ce que** l'élément de maintien tubulaire **(2)** est placé entièrement dans l'endoprothèse vasculaire **(1)** (élément de maintien interne **(2)**).

4. Combinaison selon l'une des revendications 1 ou 3, **caractérisée en ce que** l'extrémité distale, un autre élément de maintien **(2)** est placé à l'extérieur sur l'endoprothèse vasculaire **(1)** (élément de maintien externe **(2)**).

5. Combinaison selon la revendication 1 ou 2, **caractérisée en ce que** l'élément de maintien tubulaire **(2)** sort de l'endoprothèse vasculaire **(1)** au-delà de l'extrémité distale.

6. Combinaison selon la revendication 5, **caractérisée en ce que** l'élément de maintien est placé à au moins 50 % à l'intérieur de l'endoprothèse vasculaire **(1)**.

7. Combinaison selon l'une des revendications précédentes, **caractérisée en ce que** l'endoprothèse vasculaire **(1)** est reliée fixement à l'élément ou aux éléments de maintien **(2)**.

8. Combinaison selon l'une des revendications précédentes, **caractérisée en ce que** l'endoprothèse vasculaire **(1)** est constituée de PTFE ou de velours double dacron.

9. Combinaison selon l'une des revendications précédentes, **caractérisée en ce que** l'endoprothèse vasculaire **(1)** est de forme conique.

10. Combinaison selon l'une des revendications précédentes, **caractérisée en ce que** le ou les éléments de maintien internes et/ou externes **(2)** sont comprimables et radialement expansibles.

11. Combinaison selon l'une des revendications précédentes, **caractérisée en ce que** le ou les éléments de maintien **(2)** est/sont fabriqué(s) pour l'essentiel dans une ou plusieurs matières synthétiques et/ou un ou plusieurs métaux du groupe constitué du fer, du titane, du niobium, du platine, ou d'un alliage d'au moins l'un de ces métaux avec au moins un autre métal et de préférence de nitinol.

12. Combinaison selon l'une des revendications précédentes, **caractérisée en ce qu'**au moins l'un des éléments de maintien est un tuteur **(2)**.
